(19) Europäisches Patentamt
European Patent Office
Office européen des brevets

(11) **EP 4 008 322 B1**

(12) **EUROPÄISCHE PATENTSCHRIFT**

(45) Veröffentlichungstag und Bekanntmachung des Hinweises auf die Patenterteilung:
**27.11.2024 Patentblatt 2024/48**

(21) Anmeldenummer: **21175796.8**

(22) Anmeldetag: **25.05.2021**

(51) Internationale Patentklassifikation (IPC):
***A61K 9/00*** (2006.01)    ***A61K 9/50*** (2006.01)
***A61K 9/51*** (2006.01)

(52) Gemeinsame Patentklassifikation (CPC):
**A61K 9/5015; A61K 9/0019; A61K 9/5089; A61K 9/5192**

(54) **LECITHIN-PARTIKEL IM NANOMETER BEREICH ALS WIRKSTOFFTRÄGER ZUR PARENTERALEN VERABREICHUNG**

NANOMETER SIZED LECITHIN PARTICLES FOR PARENTERAL ADMINISTRATION

PARTICULES DE LÉCITHINE DE L'ORDRE DE NANOMÈTRE COMME SUPPORT DE PRINCIPE ACTIF DESTINÉES À L'ADMINISTRATION PARENTÉRALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.05.2020 EP 20176364**

(43) Veröffentlichungstag der Anmeldung:
**08.06.2022 Patentblatt 2022/23**

(73) Patentinhaber: **leon-nanodrugs GmbH 82152 Planegg (DE)**

(72) Erfinder:
• **MÜHLHÖLZL-ODÖRFER, Kathrin
80469 München (DE)**
• **HORSTKOTTE, Elke
81245 München (DE)**

(74) Vertreter: **Synergy IP Group AG
Leonhardsgraben 52
Postfach
4001 Basel (CH)**

(56) Entgegenhaltungen:
**WO-A1-2016/146516    WO-A2-00/61275
DE-A1- 10 124 952    DE-A1- 102005 053 862
US-A- 4 908 154**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Parenteralia sind sterile pharmazeutische Zubereitungen, die zur Applikation in menschliches oder tierisches Gewebe durch Injektion, Infusion oder Implantation bestimmt sind. Solche Produkte müssen frei von größeren Feststoffen sein.

Die parenterale Verabreichung lipophiler, schwer wasserlöslicher Wirkstoffe ist schwierig, da sich diese Substanzen meist nicht in der notwendigen Konzentration in Wasser lösen und Parenteralia schwer wasserlöslicher Wirkstoffe somit nicht frei von Feststoffen wären. Dann müssen Hilfsstoffe als Lösevermittler zugesetzt werden, um die wässrige Löslichkeit des Wirkstoffs zu verbessern, zum Beispiel organische Lösungsmittel oder Tenside oder Hilfslösemittel (co-solvents), wie zum Beispiel Tween 80, Tween 20, Poloxamer, Propylenglycol, Glycerin, Ethanol, Polyethylenglycol, Sorbitol, Dimethylacetamid, Cremophor EL (Pramanick, S., Singodia, D., & Chandel, V. (2013). Excipient Selection In Parenteral Formulation Development. Pharma Times, 45(3), 65-77; Bittner, B. & Mountfield, R. J., Intravenous administration of poorly soluble new drug entities in early drug discovery: the potential impact of formulating on pharmacokinetic parameters. Curr. Opinion Drug Discov. Dev. 5, 59-71 (2002)). Insbesondere Cremophor EL kann nach parenteraler Verabreichung zu anaphylaktischen Reaktionen führen (Theis, J. G. et al. Anaphylactoid reactions in children receiving high-dose intravenous cyclosporine for reversal of tumor resistance: the causative role of improper dissolution of cremophor EL. J. Clin. Oncol. 13, 2508-2516 (1995); O'Dwyer, P. J. & Weiss, R. B. Hypersensitivity reactions induced by etoposide. Cancer Res. 68, 959-961 (1984)). Co-Solvents - z.B. Alkohole - sind insbesondere bei Anwendung von parenteralen Produkten an Kindern unerwünscht.

Andere Hilfsstoffe können zu Schmerzen bei der Injektion (Duma, R. J., Akers, M. J. & Turco, S. J. in Pharmaceutical Dosage Forms: Parenteral Medications 2nd edn Vol. 1 (eds Avis, K. E., Lieberman, H. A. & Lachman, L.) 17-58 (Marcel Dekker, New York, 1992)) oder zur Ausfällung des Wirkstoffs nach der Verdünnung der injizierten Lösung im Blut führen. Wirkstoffausfällung nach Applikation kann eine lokale Schädigung der Gefäßwand durch die oft scharfkantigen Wirkstoffkristalle (Phlebitis) zur Folge haben (Ward, G. H. & Yalkowsky, S. H. Studies in Phlebitis. VI. Dilution-induced precipitation of amiodarone HCl. J. Parenter. Sci. Technol. 47, 161-165 (1993). Davio, S. R. et al. Precipitation of the renin inhibitor ditekiren upon iv infusion; in vitro studies and their relationship to in vivo precipitation in the cynomolgus monkey. Pharm. Res. 8, 80-83 (1991)).

Weiterhin werden Cyclodextrine als Lösungsvermittler für lipophile Substanzen verwendet. Diese sind selbst gut wasserlöslich und besitzen die Fähigkeit, unpolare Wirkstoffe in ihrem Inneren einzuschließen. Auf diese Art sind feststofffreie, pharmazeutische Formulierungen möglich, in denen lipophile Wirkstoffe in größeren Konzentrationen komplexiert vorliegen (zum Beispiel der Wirkstoff Carfilzomib in Kyprolis™). Nachteilig sind die hohen Kosten für diese besonderen Hilfsstoffe.

Durch Überführung des schwer wasserlöslichen Wirkstoffs in Nanopartikel kann auf problematische Hilfsstoffe in hoher Konzentration verzichtet werden und so deren Nebenwirkungen verhindert werden.

Darüber hinaus kann das Infusionsvolumen für die Verabreichung einer gleichen Dosis verringert werden, da die Wasserlöslichkeit des Wirkstoffes überschritten werden kann und so höhere Wirkstoffkonzentrationen in wässrigem Medium generiert werden können.

Bei begrenzter chemischer Stabilität in wässrigem Medium schützt eine Verkapselung in Nanopartikel den Wirkstoff sehr effizient vor unerwünschten chemischen Reaktionen. Dies kann die Produkt-Stabilität erhöhen und z.B. eine Produkt-Lagerung bei Raumtemperatur anstelle bei Kühlung ermöglichen. Außerdem kann die Lagerung der pharmazeutischen Formulierung als flüssige Suspension ermöglicht werden und die Notwendigkeit der Überführung in ein Pulver zur Rekonstitution (ready-to-use, "RTU") entfallen: ein chemisch labiler Wirkstoff muss also nicht als Pulver formuliert werden, welches vor Verabreichung resuspendiert oder gelöst werden muss, um die chemische Stabilität während der Lagerung zu gewährleisten; in diesem Fall kann der Wirkstoff als Fertigarzneimittel verabreicht werden. Dadurch wird das generelle Risiko von Medikationsfehlern durch Handhabungsfehler bei der Verdünnung oder Rekonstitution und Verdünnung vermieden. Die wichtigsten potenziellen klinischen Vorteile durch Überführung des schwer wasserlöslichen Wirkstoffes in Nanopartikel sind also:

1. Verringerung der erforderlichen Menge Lösungs-vermittelnder Tenside und damit Verringerung der Toxizität durch Lösevermittler wie z.B. Cremophor EL;
2. Kein Bedarf für Lösungs-vermittelnde co-solvents, wie z.B. organische Lösungsmittel;
3. Verringerung von Injektionsvolumina, da die Konzentration von lipophilen Wirkstoffen im wässrigem Medium (über die Sättigungslöslichkeit) hinaus erhöht werden kann;
4. die Möglichkeit, die Pharmakokinetik und -dynamik des Wirkstoffes bzw. des Arzneimittels zu verändern: Höhere Dosierungen können durch Minderung der Nebenwirkungen u.U. besser toleriert werden, niedrigere Dosen führen zu vergleichbarer Effektivität, Behandlungszyklen können angepasst werden;

(Wong, J., Brugger, A., Khare, A., Chaubal, M., Papadopoulos, P., Rabinow, B. E., Ning, J. (2008). Suspensions for intravenous (IV) injection: a review of development, preclinical and clinical aspects. Advanced Drug Delivery Reviews, 60(8), 939-954).

Die Größe der erzeugten Partikel steuert wesentlich deren Eigenschaften und ihr biologisches Verhalten und ist daher ein kritisches Qualitätsmerkmal. Die Literatur zeigt, dass es insbesondere bei soliden Tumorerkrankungen durch die Durchlässigkeit der Blutgefäße im Tumor zu einer Anreicherung von Wirkstoffen, welche als Nanopartikel verabreicht werden, kommen kann bzw. kommt (Fang, J.; Nakamura, H.; Maeda, H. The EPR Effect: Unique Features of Tumor Blood Vessels for Drug Delivery, Factors Involved, and Limitations and Augmentation of the Effect. Adv. Drug Delivery Rev. 2011, 63, 136-151. Rabinow, B. E., Nanosuspensions in drug delivery. Nature Reviews. Drug Discovery, 3(9), 785-796 (2004)). Dieses Phänomen wird als passives "drug targeting" bezeichnet. Dies erhöht die Effizienz der Therapie und ermöglicht u.U. den Einsatz von geringeren Wirkstoffdosen. Die optimale Partikelgröße ist mit 50 bis 200 nm beschrieben.

Somit ist es wichtig, die Partikelgröße einstellen zu können, denn passiver Wirkstofftransport wird so erst möglich und eine Partikelgröße von kleiner als 200 nm ist notwendig, um Filtration mit einer Porengröße von 0,22 $\mu$m als terminales Sterilisationsverfahren zu ermöglichen, insbesondere für hitzeempfindliche Wirk-und Hilfsstoffe, die nicht über thermische Methoden, wie z.B. Autoklavierung, sterilisiert werden können.

Die Geschwindigkeit der Freisetzung des Wirkstoffes aus einer partikulären Formulierung ins Blut (oder Gewebe) bestimmt unter anderem die Pharmakokinetik und -dynamik des Arzneimittels. Für die Geschwindigkeit der Freisetzung des Wirkstoffes ist neben der Hilfsstoffkomposition auch die Partikelgröße relevant. Mit fallender Partikelgröße nimmt im Vergleich die Oberfläche zu. Dies führt im Vergleich zu einer rascheren Auflösung der Partikel und somit zu einer rascheren Wirkstoff-Freisetzung.

Um die Partikelgröße von pharmazeutischen Wirkstoffen zu reduzieren, schlägt die EP 2 395 978 B1 eine so genannte Lösemittel-/Nichtlösemittelausfällung vor. Lösemittel-/Nichtlösemittelausfällung bedeutet, dass eine Substanz in einem Lösungsmittel gelöst wird und als Flüssigkeitsstrom mit einem zweiten Flüssigkeitsstrom frontal kollidiert, wodurch die gelöste Substanz ausgefällt wird. Diese sogenannte Jet-Impingement erzeugt eine hochturbulente Mischungszone aus Lösungsmittel und Nichtlösemittel und ist geeignet für die Ausfällung von Partikeln im Nanometerbereich. Die EP 2 395 978 B1 beschreibt die Lösemittel-/Nichtlösemittelausfällung in Gegenwart von oberflächenaktiven Molekülen unter Verwendung eines Jet Impingement Reaktors, in diesem Fall eines Mikrojetreaktors nach EP 1 165 224 B1. Ein solcher Reaktor weist mindestens zwei einander gegenüberliegende Düsen mit jeweils einer zugehörigen Pumpe und Zuleitung zum Verspritzen zweier flüssiger Medien auf, wobei die Flüssigkeitsströme an einen gemeinsamen Kollisionspunkt in einer Reaktorkammer frontal kollidieren. Eine Öffnung ist vorgesehen, um die entstehende Suspension aus der Reaktorkammer zu führen.

WO 2016/146516 A1 offenbart ein Herstellverfahren von Nanopartikeln. Es besteht aus dem Bereitstellen eines Lösungsmittelstromes, enthaltend ein organisches Lösungsmittel, ein Glycerophospholipid (bevorzugt Phosphatidylcholine) und eines Wirkstoffs. Nach frontaler Kollision mit einem wässrigen Strom bei hohem Druck resultieren Nanopartikel einer Größe von 170 nm.

Die Entwicklung von nanopartikulären Formulierungen zur parenteralen Verabreichung ist spezifisch auf Wirkstoff, Indikation, Dosis und die jeweilige Verabreichungsart zugeschnitten. Die Entwicklung ist Zeit- und Kostenaufwändig. Daher besteht der Bedarf für ein verbessertes Verfahren zur Herstellung von Träger-Nanopartikeln, in die eine schwer wasserlöslicher aktiver pharmazeutischer Wirkstoff (active pharmaceutical ingredient API) eingebettet werden kann, welches zudem erlaubt, die Partikelgröße in einfacher Weise einzustellen und insbesondere die Herstellung von Partikeln kleiner 0,22$\mu$m ermöglicht.

Diese Aufgabe wird durch ein Verfahren nach Anspruch 1 gelöst. Demnach wird durch die Erfindung ein Verfahren zur Herstellung eines Trägersystems bereitgestellt, wobei das Trägersystem einen pharmazeutischen Wirkstoff und mindestens ein Glycerophospholipid enthält und wobei das Verfahren die Schritte umfasst:

- Bereitstellen eines Lösungsmittelstromes, enthaltend ein organisches Lösungsmittel, das mindestens eine Glycerophospholipid und den pharmazeutischen Wirkstoff und u.U. ein oberflächen-aktives Agens (Tensid),
- Bereitstellen eines wässrigen Stromes enthaltend u.U. ein oberflächen-aktives Agens (Tensid), und
- Mischen des Lösungsmittelstromes und des wässrigen Stromes durch frontales Kollidieren, wodurch das Trägersystem als Nanopartikel ausgefällt wird,

dadurch gekennzeichnet, dass
die durch dynamische Lichtstreuung (DLS) bei einer Temperatur von 25 °C gemessene durchschnittliche Partikelgröße der ausgefällten Nanopartikel 10 bis 500 nm beträgt, der Lösungsmittelstrom 1 bis 1000 mg/mL des mindestens einen Glycerophospholipides enthält und der hydrodynamische Druck in dem wässrigen Strom mindestens 1 bar beträgt.

Die Erfinder haben ein zeit- und kostensparendes Verfahren erfunden, bei dem sich Glycerophospholipide als Hauptbestandteile eines Trägersystemes für Wirkstoffe (insbesondere wasserunlösliche) einsetzen lassen. Glycerophospholipide sind auch bei parenteraler Applikation unbedenklich und lassen sich in hohen Konzentrationen einsetzen.

Auch wasserunlösliche Wirkstoffe lassen sich durch das Verfahren in Konzentrationen oberhalb der wässrigen Sättigungslöslichkeit formulieren. Es ist durch das Verfahren ferner möglich, eine gewünschte Partikelgröße durch Erhöhung des hydrodynamischen Druckes zu erzeugen. Dadurch wird Sterilfiltration sowie passiver Wirkstofftransport ermöglicht. Durch Einbettung in eine Lipid-Matrix können durch das erfindungsgemäße Verfahren chemisch labile Wirkstoffe vor Degradation geschützt werden. Vorteilhaft ist insbesondere, dass das Verfahren für verschiedenste Wirkstoffe als ein Standardherstellungsverfahren einsetzbar ist. Durch die Wahl der Partikelgröße sowie weiterer Bestandteile des Trägersystems lässt sicher ferner auch die Freisetzung des Wirkstoffes aus dem Trägersystem verändern. Die Freisetzung des Wirkstoffes kann durch Verkleinerung der Partikel beschleunigt werden. Die Partikelgröße kann über den hydrodynamischen Druck eingestellt werden. Die Inkorporation von Wirkstoff führt nur zu einer geringfügigen Zunahme der Partikelgröße, der Zusammenhang zwischen Steigerung des Drucks und Abnahme der Partikelgröße bleibt erhalten und beeinflusst die Partikelgröße entscheidend.

Durch das Mischen des Lösungsmittelstromes und des wässrigen Stromes wird eine Suspension erhalten, in der das Trägersystem als Nanopartikel ausgefällt vorliegt. Das Trägersystem ist ein Feststoff, der einen pharmazeutischen Wirkstoff, mindestens ein Glycerophospholipid und gegebenenfalls weitere Inhaltsstoffe, z.B. Cholesterol oder eines seiner Derivate, enthält und in Form von Nanopartikel vorliegt. Die Tenside sind nicht Bestandteil des festen Partikels, sondern liegen in gelöstem Zustand in der Suspension bzw. benetzend auf der Oberfläche des Partikels vor.

Der pharmazeutische Wirkstoff ist insbesondere eine niedermolekulare Verbindung (ein sogenanntes "kleines Molekül" bzw. "small molecule") oder ein Peptid. Der Begriff pharmazeutische Wirkstoff umfasst aber auch Antikörper, RNA, Proteine etc.. In einer Ausführungsform ist der pharmazeutische Wirkstoff wasserunlöslich. Ein wasserunlöslicher pharmazeutischer Wirkstoff hat eine Löslichkeit von < 1 mg/ml ("very slightly soluble") und präferiert von < 0,1 mg/ml ("practically insoluble") in Wasser. Das Verfahren eignet sich für wasserunlösliche Wirkstoffe. Der Begriff das "mindestens eine Glycerophospholipid" umfasst ein oder mehrere Glycerophospholipide. Der Begriff "Glycerophospholipide" (Phosphoglyceride, Phosphoglycerolipide) umfasst Phosphatidylcholine (Lecithine), Phosphatidylethanolamine, Phosphatidylserine und Phosphatidylinositole.

Phosphatidylcholine (Lecithine) sind als das mindestens eine Glycerophospholipid besonders bevorzugt. Phosphatidylcholin oder Lecithin (z. B. Lipoid S 100) Lipoid S 100 ist beispielsweise ein geeignetes, kommerziell erhältliches Glycerophospholipid.

Das organische Lösungsmittel kann ein organisches Lösungsmittel oder ein Gemisch aus mehreren, z.B. zwei, organischen Lösungsmitteln sein. Vorzugsweise ist das organische Lösungsmittel / sind die organischen Lösungsmittel mit Wasser mischbar. Werden mehrere organische Lösungsmittel eingesetzt, sind diese miteinander mischbar.

Die Hauptkomponente (z.B. ≥ 50, 60, 70, 75, 80, 90, 95 oder 99 Gew.-%) des wässrigen Stroms ist Wasser. Der wässrige Strom besteht aus Wasser und enthält wahlweise Hilfsstoffe wie z.B. Tenside, Puffer, etc..

Der Begriff Nanopartikel bezeichnet einen Feststoff in Form von Partikeln mit einer durchschnittlichen Partikelgröße zwischen 1 und 1000 nm.

Das Mischen des Lösungsmittelstromes und des wässrigen Stromes umfasst ein Kollidieren der Ströme. Vorzugsweise sind die Ströme in einem Winkel von etwa 180 °C zueinander angeordnet. Mit anderen Worten, die Ströme treffen frontal zusammen.

Erfindungsgemäß kann die durchschnittliche Partikelgröße durch dynamische Lichtstreuung (DLS) (z.B. mit einem Malvern Zetasizer AT/Advance Range/ZS90/ZS oder Malvern Ultra von Malvern Instruments Ltd.; Nanophox u.a. DLS Messgeräten von Wyatt Technology Corporation; Litesizers von Anton Paar GmbH) bei einer Temperatur von 25 C° gemessen werden. Diese Temperatur wird während der Messung konstant gehalten. Der z-gemittelte Durchmesser wird zusammen mit dem Polydispersitätsindex (PDI) vorzugsweise aus der Kumulantenanalyse der Autokorrelationsfunktion der gemessenen DLS- Intensität, wie in ISO22412:2008 definiert, berechnet. Vorzugsweise werden die Partikelgrößen mit einem Zetasizer der Firma Malvern Instruments Ltd. gemessen.

Der PDI ist eine dimensionslose Schätzung der Breite der Partikelgrößenverteilung, skaliert von 0 bis 1. Der Begriff durchschnittliche Partikelgröße bezieht sich auf den z-gemittelten Durchmesser.

Im Allgemeinen kann der hydrodynamische Druck entweder auf theoretischer Basis berechnet oder mit einem Druckmesser überwacht werden. Die Berechnung des hydrodynamischen Druckes erfolgt unter Verwendung der folgenden Formel

$$p = \left( \frac{1}{2} * \rho * \left[ \frac{Q}{\pi * r^2} \right]^2 \right) * 10^{-5}$$

wobei

p = den Druck in dem Strom ([bar]),

$\rho$ = die Dichte der Zusammensetzung (alle Bestandteile) des Stromes ([kg/m$^3$]),
Q = die Flussrate des Stromes ([m$^3$/s]) und
r = den Radius der Düse ([m]) bezeichnet.

Die Geschwindigkeit des Stromes v (m/s) lässt sich mit folgender Formel berechnen:

$$v = \left[\frac{Q}{\pi * r^2}\right]^2$$

Die Flussrate ist eine der Variablen für die Druckberechnung. Vorzugsweise wird sichergestellt, dass die Pumpen, die die Bewegung der Ströme auslösen, vor Beginn des Produktionsprozesses funktionsfähig sind und die erforderliche Pumpleistung erfüllen. Die Flussrate kann - ohne Verwendung des Reaktors - durch eine Pumpenkalibrierung überprüft werden: Für jede Pumpe wird eine definierte Flussrate eingestellt, das Pumpen wird über die Zeit x ausgeführt und das gepumpte Volumen wird mit Hilfe einer Waage auf sein Gewicht überprüft. Dabei werden experimentelle und theoretische Gewichte verglichen.

Eine Inline-Überwachung der Flussraten kann durch die Implementierung von Flussratenmessern in den Zuführleitungen zum Reaktor durchgeführt werden, so dass die Flussraten auch während des Prozesses kontrolliert werden können. Hierfür können CORI FLOW Meter oder Coriolis-Durchflussmesser von Bronkhorst verwendet werden.

Ein wesentliches Merkmal der Erfindung ist der hydrodynamische Druck bzw. die Geschwindigkeit der aufeinandertreffenden Ströme. Wie oben erläutert, wird der hydrodynamische Druck aus der Flussrate und der Dichte der Ströme (Strahlen) sowie aus dem Radius der Düsen, durch die die Ströme in die Reaktorkammer (Kammer, in der die beiden Ströme gemischt werden) ausgestoßen werden, berechnet. Um einen erfindungsgemäßen hydrodynamischen Druck zu erreichen, ist es im Allgemeinen bevorzugt, dass die Flussrate des Lösungsmittelstromes 1 ml/min bis 10 l/min, bevorzugter 10 ml/min bis 1,33 l/min, am meisten bevorzugt 50 ml/min bis 250 ml/min, beträgt und die Flussrate des wässrigen Stromes 1 ml/min bis 10 l/min, bevorzugter 10 ml/min bis 1,33 l/min, am meisten bevorzugt 50 ml/min bis 250 ml/min beträgt. Es wird bevorzugt, dass die Geschwindigkeit des Lösungsmittelstromes 2 bis 150 m/s, vorzugsweise 10 bis 125 m/s, und die Geschwindigkeit des wässrigen Stromes 2 bis 150 m/s, vorzugsweise 20 bis 125 m/s, beträgt.

Die Dichte des Lösungsmittelstromes beträgt vorzugsweise 0,600 bis 1,300 g/mL und die Dichte des wässrigen Stromes beträgt vorzugsweise 0,600 bis 1,300 g/mL. Der Durchmesser (Radius = halber Durchmesser) der Düsen sollte vorzugsweise 5 bis 5.000 $\mu$m, vorzugsweise 10 bis 3.000 $\mu$m, noch bevorzugter 10 bis 2.000 $\mu$m und am meisten bevorzugt 50 bis 500 $\mu$m, betragen.

Vorzugsweise beträgt der Abstand zwischen den gegenüberliegenden Düsen (für die beiden Ströme) weniger als 5 cm, vorzugsweise weniger als 3 cm und vorzugsweise weniger als 1 cm.

Somit ergeben sich, in Abhängigkeit der Wahl des Düsendurchmessers und des gewählten Mischungsverhältnisses, hydrodynamische Drücke für den Lösungsmittelstrom von >0,02 bar, vorzugsweise von >0,5 bar, noch bevorzugter von >10 bar und am meisten bevorzugt von >30 bar. Es ergeben sich, in Abhängigkeit der Wahl des Düsendurchmessers, hydrodynamische Drücke für den wässrigen Strom von >0,02 bar, vorzugsweise von >0,5 bar, noch bevorzugter von >10 bar und am meisten bevorzugt von >30 bar. Vorzugsweise ist der hydrodynamische Druck im Lösungsmittelstrom gleich oder kleiner als der hydrodynamische Druck des wässrigen Stroms.

Die Reaktorkammer (Kammer, in der die beiden Ströme kollidieren und gemischt werden) kann mit Gas beaufschlagt werden. Gas, insbesondere Inertgas oder Inertgasgemische, aber auch reaktives Gas kann durch einen Gaseinlass in die Reaktorkammer zugeführt werden. Vorzugsweise wird der Prozess ohne Gas durchgeführt. Der Gasdruck in der Reaktorkammer sollte vorzugsweise 0,05 bis 30 bar, vorzugsweise 0,2 bis 10 bar und besonders bevorzugt 0,5 bis 5 bar betragen.

Vorzugsweise ist das Verfahren ein kontinuierliches Verfahren.

Vorzugsweise wird die durch das Mischen erhaltene Suspension bzw. das darin enthaltene Trägersystem weiterverarbeitet, vorzugsweise zu einer pharmazeutischen Zusammensetzung. Dies kann einen oder mehrere Schritte umfassen, exemplarisch die Entfernung des organischen Lösungsmittels, das Einstellen des pH Wertes, das Einstellen der Pufferkapazität, die Zugabe von AntiOxidantien, das Einstellen der Osmolalität, die terminale Sterilisation durch Sterilfiltration, die Gefriertrocknung, die Abfüllung usw..

In einer Ausführungsform wird die durch das Mischen erhaltene Suspension oder eine Suspension, enthaltend das Trägersystem, einer Sterilfiltration unterzogen. Das Filtermaterial wird beispielsweise aus Celluloseacetat, regenerierter Cellulose, Polyamid, Polyethersulfon (PES), modifiziertem Polyethersulfon (mPES), Polyvinylidenfluorid (PVDF) und Polytetrafluorethylen (PTFE) ausgewählt. Die Sterilfiltration ermöglicht die parenterale Anwendung des Produkts und ist - entsprechend den gesetzlichen Anforderungen - die Methode der Wahl für Produkte zur parenteralen Anwendung, die nicht durch feuchte oder trockene Hitze endsterilisiert werden können. Dank dem erfindungsgemäßen Verfahren kann die Partikelgröße genau eingestellt werden. Dies ist wichtig, da der Verlust an Feststoff bei der Filtration minimiert werden kann, wenn die Nanopartikel eine durchschnittliche Partikelgröße unter 210 nm (z.B. 150 nm) und einen niedrigen PDI aufweisen (bei Wahl eines geeigneten Filtermaterials).

Die Verben "umfassen" und "enthalten" und ihre Konjugationen umfassen den Begriff "bestehen aus" und seine Konjugationen. Die Begriffe "ein" oder "eine" umfassen den Begriff "mindestens ein(e)".

Ausführungsformen der Erfindung können in beliebiger Weise kombiniert werden, es sei denn, dies ist eindeutig nicht beabsichtigt. Bevorzugte Ausführungsformen der Erfindung werden auch in den abhängigen Ansprüchen angegeben. Vorzugsweise beträgt die durchschnittliche Partikelgröße der ausgefällten Nanopartikel 25 bis 500 nm, bevorzugt 50 bis 250 nm, oder 70 bis 170 nm, oder 80 bis 150 nm, oder 90 bis 110 nm.

Insbesondere mit den nachfolgenden hydrodynamischen Drücken werden die obigen durchschnittlichen Partikelgrößen erreicht:

Erfindungsgemäß beträgt der hydrodynamische Druck in dem wässrigen Strom mindestens 1 bar, besonders bevorzugt 2 bar bis 60 bar.

Vorzugsweise beträgt der hydrodynamische Druck in dem Lösungsmittelstrom mindestens 0.01 bar, bevorzugt mehr als 0.1 bar, bevorzugter mehr als 1 bar, am bevorzugtesten 2 bar bis 60 bar.

Vorzugsweise wird die durchschnittliche Partikelgröße der ausgefällten Nanopartikel durch den hydrodynamischen Druck in dem wässrigen Strom und/oder den hydrodynamischen Druck in dem Lösungsmittelstrom eingestellt, wobei durch einen höheren hydrodynamischen Druck Nanopartikel geringerer durchschnittlicher Partikelgröße ausgefällt werden. Dies gilt insbesondere bei konstantem Mischungsverhältnis von wässrigem Strom und Lösungsmittelstrom. Vorzugsweise ist das mindestens eine Glycerophospholipid ausgewählt aus Phosphatidylcholinen.

Erfindungsgemäß enthält der Lösungsmittelstrom 1 bis 1000 mg/mL, bevorzugt 50 bis 700 mg/mL, 100 bis 500 mg/mL, 100 bis 300 mg/mL, zum Beispiel etwa 150 oder etwa 225 mg/mL, des mindestens einen Glycerophospholipides. Der Gehalt des mindestens einen Glycerophospholipides ist für die Bildung des Trägersystems, die Stabilisierung und die Freisetzung des Wirkstoffs wichtig.

Vorzugsweise enthält der Lösungsmittelstrom und/oder der wässrige Strom 1 bis 250 mg/mL, bevorzugt 5 bis 100 mg/mL, besonders bevorzugt 8 bis 50 mg/mL, eines Steroides, vorzugsweise Cholesterol oder eines seiner Derivate. Der Gehalt an Cholesterol oder eines seiner Derivate kann die Bildung sehr kleiner Trägersysteme unterstützen (siehe z.B. Versuchsteil). Dabei kann auch bevorzugt sein, den pH-Wert des wässrigen Stromes auf 5 bis 2,5 einzustellen, insbesondere, wenn das Cholesterolderivat in dem wässrigen Strom enthalten ist. Besonders bevorzugt ist Desoxycholsäure und/oder Natriumdesoxycholat als Cholesterolderivat.

Vorzugsweise umfasst das Mischen des Lösungsmittelstromes und des wässrigen Stromes ein Mischen in einem Volumenverhältnis von Lösungsmittelstrom zu wässrigem Strom von zwischen 1:1 bis 1:20, bevorzugter von zwischen 1:1 bis 1:10.

Optional enthält der wässrige Strom 0.1 bis 250 mg/mL, bevorzugt 0.5 bis 100 mg/mL, besonders bevorzugt 1.0 bis 15 mg/mL, eines oder mehrerer ionischer oder nichtionischer Tenside, wobei das Tensid vorzugsweise Natriumoleat und/oder Ölsäure ist.

In einer Ausführungsform enthält der Lösungsmittelstrom und/oder der wässrige Strom ferner ein oder mehr Tenside ausgewählt aus polyethoxyliertem Rizinusöl (Kolliphor ELP), Macrogol 15 Hydroxystearat (Kolliphor HS15), pegyliertem Rizinusöl, Docusate-Natrium, Poly(ethylenglycol)-*block*-poly(propylenglycol)-*block*-poly(ethylenglycol) (Poloxamer 188), Vitamin E und Derivate davon, Polysorbat 20 (Tween 20), Polysorbat 40 (Tween 40), Polysorbat 60 (Tween 60), Polysorbat 80 (Tween 80). Das Tensid kann die Bildung des Trägersystems beeinflussen, das Trägersystem stabilisieren und die Freisetzung des pharmazeutischen Wirkstoffs beeinflussen.

Vorzugsweise ist das organische Lösungsmittel ein mit Wasser mischbares Lösungsmittel, wobei das organische Lösungsmittel vorzugsweise i) Ethanol oder ii) eine Mischung von Ethanol mit einem weiteren organischen Lösungsmittel, das mit Wasser und Ethanol mischbar ist, oder iii) Aceton ist. Der Ethanol kann reiner Ethanol sein oder wässriger Ethanol, d.h. Ethanol mit einem bestimmten Wassergehalt (z.B. 85 Vol.-%). Vorzugsweise ist das weitere organische Lösungsmittel Aceton.

In einer bestimmten Ausführungsform enthält der wässrige Strom 1 bis 1000 mM (Säure plus konjugierte Base), bevorzugt 20 bis 500 mM oder 20 bis 250 mM (Säure plus konjugierte Base), eines Puffersystems.

Vorzugsweise ist die Konzentration (mg/mL) an pharmazeutischen Wirkstoff in dem Lösungsmittelstrom wesentlich geringer als die Konzentration (mg/mL) des Glycerophospholipides. Da beide Komponenten, Wirkstoff sowie Trägermaterial (Glycerophospholipid), co-präzipitieren, bestehen die erzeugten Partikel aus wesentlich mehr Trägermaterial als Wirkstoff. Die Eigenschaften der erzeugten wirkstoffhaltigen Partikel werden daher wesentlich durch das Trägermaterial determiniert.

Vorzugsweise enthält der Lösungsmittelstrom 0,25 bis 250 mg/mL, bevorzugt 1 bis 150 mg/mL, besonders bevorzugt 10 bis 100 mg/mL, zum Beispiel etwa 50 mg/mL, des pharmazeutischen Wirkstoffes. Der Gehalt an pharmazeutischem Wirkstoff im Lösungsmittelstrom kann für die Bildung des Trägersystems, die Stabilisierung und die Freisetzung des Wirkstoffs wichtig sein.

In einer Ausführungsform wird ein Jet Impingement Reaktor zur Durchführung des Verfahrens verwendet. Ein Beispiel eines solchen Reaktors ist aus EP 1 165 224 B1 bekannt. Es können auch andere Mikromischvorrichtungen und/oder Ausfällungsvorrichtungen mit kollidierenden Strömen verwendet werden, z.B. eine Vorrichtung wie in WO 2018/234217 A1 beschrieben.

In einer Ausführungsform enthält der Lösungsmittelstrom und das Trägersystem Hydroxypropylmethylcelluloseacetatsuccinat (HPMCAS). In dieser Ausführungsform ist das organische Lösungsmittel vorzugsweise Aceton. HPMCAS fällt beim Mischen mit Wasser aus und bildet somit einen Bestandteil des Trägersystemes. In dieser Ausführungsform beträgt die Konzentration an HPMCAS in dem Lösungsmittelstrom vorzugsweise 1 bis 100 mg/mL, bevorzugter 5 bis 30 mg/mL, und die Konzentration des pharmazeutischen Wirkstoffes in dem Lösungsmittelstrom beträgt vorzugsweise 1 bis 30 mg/mL, bevorzugter 2 bis 15 mg/mL. In dieser Ausführungsform beträgt der pH-Wert des wässrigen Stromes vorzugsweise 6 bis 8, bevorzugter 6 bis 7.

Die Erfindung wird nachfolgend an Hand nichtbeschränkender Beispiele illustriert:

Beispiel 1: Herstellung wirkstofffreier Trägerpartikel (nicht erfindungsgemäß )

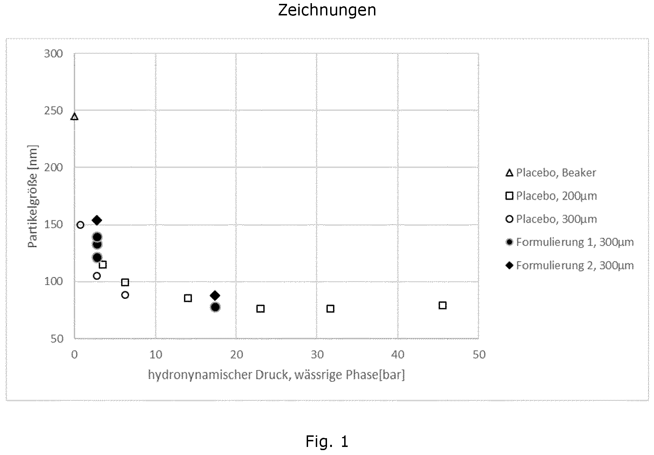

Die organische Phase enthielt 225 mg/ml Lipoid S 100 in EtOH/Aceton 1:1 (v/v). Die wässrige Phase enthielt 10,5 mg/ml Natriumoleat. Ein Teil der organischen Phase wurde mit fünf Teilen der wässrigen Phase in einem MicroJet-Reaktor mit zwei Düsen von je 200 $\mu$m (Fig. 1, ungefüllte Vierecke) oder je 300 $\mu$m (Fig. 1, ungefüllte Punkte) gemischt.

Wie Fig. 1 entnommen werden kann, werden in Abhängigkeit des gewählten hydrodynamischen Druckes Trägerpartikel zwischen 150 nm und 79 nm erhalten. Je höher der hydrodynamische Druck, desto kleiner die erhaltenen Partikel.

Die Effizienz der Mischung zeigt der Vergleich mit der manuellen Durchführung der Präzipitation durch Mischen von organischer Phase und wässriger Phase im Becherglas (Fig. 1, ungefülltes Dreieck).

Placebo-Daten sind tabellarisch in Tabelle 1 zusammengefasst.

Tabelle 1.

| Düsend urchme sser / $\mu$m | Flussrat e Lösungs mitel / ml/min | Geschw indigkeit Lösungs mittel / m/s | Druck Lösungs mittel / bar | Flussrat e Wasser / ml/min | Geschw indigkeit Wasser / m/s | Druck Wasser / bar | Partikel größe / nm | PDI |
|---|---|---|---|---|---|---|---|---|
| | | | | | | | 245 | 0,26 |
| 300 | 30 | 7,1 | 0,20 | 150 | 35,4 | 6,3 | 89 | 0,22 |
| 300 | 20 | 4,7 | 0,09 | 100 | 23,6 | 2,8 | 105 | 0,22 |
| 300 | 10 | 2,4 | 0,02 | 50 | 11,8 | 0,7 | 150 | 0,26 |
| 200 | 40 | 21,2 | 1,80 | 200 | 106,1 | 56,3 | - | - |
| 200 | 36 | 19,1 | 1,46 | 180 | 95,5 | 45,6 | 79 | 0,26 |
| 200 | 30 | 15,9 | 1,01 | 150 | 79,6 | 31,7 | 76 | 0,28 |
| 200 | 25,6 | 13,6 | 0,74 | 128 | 67,9 | 23,1 | 76 | 0,23 |
| 200 | 20 | 10,6 | 0,45 | 100 | 53,1 | 14,1 | 86 | 0,25 |
| 200 | 13,3 | 7,1 | 0,20 | 66,7 | 35,4 | 6,3 | 100 | 0,23 |
| 200 | 10 | 5,3 | 0,11 | 50 | 26,5 | 3,5 | 115 | 0,24 |

Beispiel 2: erfindungsgemäße Herstellung wirkstoffhaltiger Partikel (Formulierung 1)

Ein lipophiler, wasserunlöslicher Wirkstoff der Molmasse 400 g/mol wurde in Trägerpartikel ähnlich zu denjenigen in Beispiel 1 beschrieben inkorporiert. Der Versuch wurde wie oben mit Düsen mit einem Durchmesser von 300 $\mu$m durchgeführt, allerdings enthielt die organische Phase zusätzlich 50 mg/mL des Wirkstoffes. Die wässrige Phase enthielt 1 mg/ml Natriumoleat.

Die Inkorporation von Wirkstoff führt zu einer Zunahme der Partikelgröße von circa 30 nm im direkten Vergleich zu Beispiel 1. Es sei erwähnt, dass die Zusammensetzung von Formulierung 1 und Placebo aus Beispiel 1 sich in der Konzentration des Natriumoleats unterscheidet. Es wurden in Abhängigkeit des gewählten hydrodynamischen Druckes im wässrigen Strom Partikel zwischen 140 nm und 78 nm erhalten (Fig. 1, gefüllte Punkte). Je höher der hydrodynamische Druck, desto kleiner die erhaltenen Partikel.

Ein Teil organischer Phase wurde mit fünf Teilen wässriger Phase in einem MicroJet-Reaktor mit zwei Düsensteinöffnungen von je 300 μm gemischt. Die Daten sind tabellarisch in Tabelle 2 zusammengefasst.

Tabelle 2.

| Düsendurch messer / μm | Flussrat e Lösungs mitel / ml/min | Geschwin digkeit Lösungsmi ttel / m/s | Druck Lösungs mittel / bar | Flussr ate Wass er / ml/mi n | Geschwin digkeit Wasser / m/s | Druc k Was ser / bar | Partikelg röße / nm | P DI |
|---|---|---|---|---|---|---|---|---|
| 300 | 50 | 11,8 | 0,56 | 250 | 58,9 | 17,3 | 78 | 0,27 |
| 300 | 20 | 4,7 | 0,09 | 100 | 23,6 | 2,7 | 133 | 0,10 |
| 300 | 20 | 4,7 | 0,09 | 100 | 23,6 | 2,7 | 121 | 0,17 |
| 300 | 20 | 4,7 | 0,09 | 100 | 23,6 | 2,7 | 139 | 0,20 |

Beispiel 3: erfindungsgemäße Herstellung wirkstoffhaltiger Partikel (Formulierung 2)

Der Versuch wurde wie in Beispiel 2 durchgeführt, allerdings enthielt die wässrige Phase nicht 1 mg/ml Natriumoleat, sondern 17 mg/ml Natriumoleat.

Es werden in Abhängigkeit des gewählten hydrodynamischen Druckes Partikel zwischen 154 nm und 88 nm erhalten. Je höher der hydrodynamische Druck, desto kleiner die erhaltenen Partikel. (Fig. 1, gefüllte Rauten). Daten sind tabellarisch in Tabelle 3 zusammengefasst.

Tabelle 3.

| Düsendurch messer / μm | Flussrat e Lösungs mitel / ml/min | Geschwin digkeit Lösungsmittel / m/s | Druck Lösungs mittel / bar | Fluss rate Wass er / ml/mi n | Geschwin digkeit Wasser / m/s | Druc kWas ser / bar | Partikelg röße / nm | PDI |
|---|---|---|---|---|---|---|---|---|
| 300 | 50 | 11,8 | 0,56 | 250 | 58,9 | 17,4 | 88 | 0,25 |
| 300 | 20 | 4,7 | 0,09 | 100 | 23,6 | 2,8 | 154 | 0,15 |

Beispiel 4: Zusammensetzung des Produktes

Die Konzentrationen der Ausgangslösungen sowie die Konzentrationen in der Suspension für die Beispiele 1 bis 3 sind in Tabelle 4 zusammengefasst:

Tabelle 4.

| | Organische Phase EtOH/Aceton 1:1 (v/v) | | Wässrige Phase | Konzentration in Suspension in mg/ml | | |
|---|---|---|---|---|---|---|
| | API | Lipoid 5100 | NaOleat | API | Lipoid S100 | NaOleat |
| | mg/ml | mg/ml | mg/ml | mg/ml | mg/ml | mg/ml |
| Placebo | 0 | 225 | 10,5 | 0 | 37,5 | 8,75 |
| Formulierung 1 | 50 | 225 | 1 | 8,3 | 37,5 | 0,8 |
| Formulierung 2 | 50 | 225 | 17 | 8,3 | 37,5 | 14,2 |

Die Konzentration des Wirkstoffes ist deutlich geringer als die des Trägermateriales Lecithin. Der theoretische Wirkstoffgehalt der Partikel ist 18,2%. Die erzeugten Partikel bestehen somit aus wesentlich mehr Trägermaterial als Wirkstoff.

Beispiel 4: Lösungsmittelentzug mittels cross flow filtration CFF am Beispiel von Formulierung 2 aus Beispiel 3

CFF Experimente wurden mit einer PES 100 kDa Hohlfaser von Repligen und fünf Waschvolumina durchgeführt. Als Waschmedium wurde Wasser unter Zusatz von 10 mg/mL bzw. 5 mg/mL Natriumoleat benutzt.

Formulierung 2 wurde für diese Versuche mit 50 ml/min Flussrate für die organische Phase und mit einem Düsendurchmesser von 300 μm hergestellt. Partikelgrößen von unter 90 nm wurden erzielt. Die Abtrennung des Lösemittels mittels CFF war möglich ohne Verlust an Wirkstoff, sofern die Suspension 1:1 vorverdünnt wurde vor CFF und mit einer 10 mg/ml Natriumoleat gewaschen wurde. Wurde kein Verdünnungsschritt eingefügt oder mit einer geringeren Konzentration von Surfactant gearbeitet, kam es zu Wirkstoff-Verlusten, wie in Tabelle 5 gezeigt.

Tabelle 5.

| ID | [NaOleat] in CFF Waschmedium | Pre-CFF | | Post-CFF | |
|---|---|---|---|---|---|
| | | PS nm | [API] (mg/mL) | PS nm | [API] (mg/mL) |
| 2 | 10 mg/mL | 87,8 | 8,2 | 86,4 | 5,5 |
| 3 | 10 mg/mL | 85,1 | 4,0 | 82,7 | 5,4 |
| 4 | 5 mg/mL | | | 88,5 | 3,2 |

Beispiel 5: Sterile Filtration der Formulierung aus Beispiel 4

ID 3 aus Beispiel 4 wurde in Hinsicht auf Steril-Filtration getestet. Sowohl mit PES als auch PVDF Material konnten hohe Wiederfindungswerte erzielt werden (siehe Tabelle 6).

Tabelle 6.

| Filter | Wirkstoffgehalt vor Filtration (mg/mL) | Wirkstoffgehalt nach Filtration (mg/mL) | Recovery % |
|---|---|---|---|
| CA 0,22 μm | 5,65 | 5,17 | 91,50 |
| PES 0,22 μm | | 5,58 | 98,76 |
| PTFE 0,22 μm | | 5,34 | 94,51 |
| PVDF 0,22 μm | | 5,57 | 98,58 |
| Nylon 0,22 μm | | 5,17 | 91,50 |

Beispiel 6: Stabilitätsdaten

Formulierung 3 wurde einer initialen Stabilitätsprüfung bei Raumtemperatur unterzogen. Die Suspension zeigte sowohl physikalische als auch chemische Stabilität im Beobachtungszeitraum von 1 Monat (siehe Tabelle 7).

Tabelle 7.

| | T=0 | T=1M |
|---|---|---|
| Partikelgröße [nm] | 106,9 | 106,5 |
| PDI | 0,29 | 0,28 |
| pH | 7,5 | 7,5 |
| Gehalt [mg/ml] | 5,65 | 5,71 |

Beispiel 7:

Weitere Nanopartikel mit einem anderen Wirkstoff wurden erfindungsgemäß hergestellt. Der Wirkstoff war ein Peptid, bestehend aus 20 Aminosäuren, mit einem Gewicht von 2180 Dalton. Das Peptid ist in 10 mg/mL bei pH 3,5 wasserlöslich.

Eine Lösung mit 50 mg/mL des Wirkstoffes und 150 mg/mL Lipoid S 100 wurde in einem Gemisch (Solvent) von Ethanol (85 Vol.-%) und Wasser (15 Vol.-%) hergestellt. Diese Lösung wurde in einem Strom mit einem wässrigen Strom (Non-Solvent) mit verschiedenen Mengen an Natriumdesoxycholat sowie gegebenenfalls Salzsäure kollidiert. Tabelle 8 zeigt die Bedingungen:

Tabelle 8.

| Formulierung | Konzentration im Lösungsmittelstrom (85: 15 EtOH:H2O) [mg/mL] | | Konzentration im Non-Solvent [mg/mL] | | Verhältnis Solvent/Non-Solvent |
|---|---|---|---|---|---|
| | Wirkstoff | Lipoid S 100 | Non-Solvent | Natriumdesoxycholat | |
| 359 | 50 | 150 | HCl pH3.5 | 25,0 | 0,5 |
| 385 | | | HCl pH3.5 | 10,0 | 0,5 |
| 414 | | | HCl pH3.0 | 0,5 | 0,5 |
| 415 | | | HCl pH3.0 | 1,0 | 0,5 |
| 416 | | | HCl pH3.0 | 2,0 | 0,5 |
| 417 | | | HCl pH3.0 | 5,0 | 0,5 |
| 422 | | | HCl pH3.0 | 7,5 | 0,5 |
| 418 | | | Wasser | 0,5 | 0,5 |
| 419 | | | Wasser | 1,0 | 0,5 |
| 420 | | | Wasser | 2,0 | 0,5 |
| 421 | | | Wasser | 5,0 | 0,5 |
| 423 | | | Wasser | 7,5 | 0,5 |

Die durchschnittliche Partikelgröße und Größenverteilung wurde analysiert und die Ergebnisse sind in Tabelle 9 dargestellt:

Tabelle 9.

| Formulierung | durchschnittliche Partikelgröße [nm] | PDI |
|---|---|---|
| 359 | 211,7 | 0,180 |
| 385 | 214,8 | 0,200 |
| 414 | 438,4 | 0,140 |
| 415 | 487,8 | 0,273 |
| 416 | 230,5 | 0,067 |
| 417 | 557,1 | 0,224 |
| 422 | 750,3 | 0,385 |
| 418 | 723,4 | 0,319 |
| 419 | 496,9 | 0,271 |
| 420 | 537,0 | 0,273 |
| 421 | 549,9 | 0,306 |
| 423 | 605,2 | 0,508 |

Beispiel 8:

Weitere Nanopartikel mit dem Wirkstoff wie in Beispiel 5 wurden erfindungsgemäß hergestellt. Tabelle 10 zeigt die Versuchsbedingungen:

Tabelle 10.

| Formulierung | Konzentration im Solvent (85:15 EtOH:H2O) [mg/mL] | | | | Konzentration im Non-Solvent [mg/mL] | | Verhältnis Solvent/Non-Solvent |
|---|---|---|---|---|---|---|---|
| | Wirkstoff | Lipoid S 100 | Ölsäure | Natriumoleat | Non-Solvent | Natriumoleat | |
| 424 | 50 | 150 | 2,0 | - | 25.0 mg/mL Natriumdesoxycholat in Wasser | - | 0,5 |
| 425 | | | 5,0 | - | | - | |
| 426 | | | 10,0 | - | | - | |
| 427 | | | 20,0 | - | | - | |
| 428 | | | - | 2,0 | | - | |
| 429 | | | - | 5,0 | | - | |
| 430 | | | - | - | | 5,0 | |
| 431 | | | - | - | | 10,0 | |

EP 4 008 322 B1

Die durchschnittliche Partikelgröße und Größenverteilung wurde analysiert und die Ergebnisse sind in Tabelle 11 dargestellt:

Tabelle 11.

| Formulierung | durchschnittliche Partikelgröße [nm] | PDI |
|---|---|---|
| 424 | 209,2 | 0,041 |
| 425 | 115,6 | 0,131 |
| 426 | 260,0 | 0,154 |
| 427 | 548,2 | 1,000 |
| 428 | 255,5 | 0,200 |
| 429 | 546,9 | 1,000 |
| 430 | 324,0 | 0,613 |
| 431 | 338,6 | 0,012 |

Beispiel 9:

Weitere Nanopartikel mit dem Wirkstoff wie in Beispiel 5 wurden erfindungsgemäß hergestellt. Tabelle 12 zeigt die Versuchsbedingungen:

Tabelle 12.

| Formulierung | Konzentration im Solvent (85:15 EtOH:H2O) [mg/mL] | | | | Konzentration im Non-Solvent [mg/mL] | | Verhältnis Solvent/Non-Solvent |
|---|---|---|---|---|---|---|---|
| | Wirkstoff | Lipoid S100 | Ölsäure | Natriumoleat | Non-Solvent | Natriumoleat | |
| 432 | 50 | 150 | 2,0 | - | Wasser | - | 0,5 |
| 433 | | | 5,0 | - | | - | |
| 434 | | | 10,0 | - | | - | |
| 435 | | | 20,0 | - | | - | |
| 436 | | | 2,0 | - | | - | |
| 437 | | | - | 5,0 | | - | |
| 438 | | | - | - | | 5,0 | |
| 439 | | | - | - | | 10,0 | |

Die durchschnittliche Partikelgröße und Größenverteilung wurde analysiert und die Ergebnisse sind in Tabelle 13 dargestellt:

Tabelle 13.

| Formulierung | durchschnittliche Partikelgröße [nm] | PDI |
|---|---|---|
| 432 | 270,0 | 0,057 |
| 433 | 265,0 | 0,193 |
| 434 | 225,9 | 0,168 |
| 435 | 225,6 | 0,235 |
| 436 | 410,8 | 0,049 |
| 437 | 388,9 | 0,117 |
| 438 | 198,4 | 0,241 |

(fortgesetzt)

| Formulierung | durchschnittliche Partikelgröße [nm] | PDI |
|---|---|---|
| 439 | 256,7 | 0,401 |

Beispiel 10:

Weitere Nanopartikel mit dem Wirkstoff wie in Beispiel 5 wurden erfindungsgemäß hergestellt. Tabelle 14 zeigt die Versuchsbedingungen:

Tabelle 14.

| Formulierung | Konzentration im Solvent (85:15 EtOH:H2O) [mg/mL] | | Non-solvent (mg/mL) | | | | Verhältnis Solvent/Non-Solvent |
|---|---|---|---|---|---|---|---|
| | Lipoid S100 | Wirkstoff | Natriumoleat | Tween 20 | Tween 60 | Tween 80 | |
| 152 | 150 | 50 | 10 | | | 0,5 | 1:2 |
| 153 | 150 | 50 | 0 | | | 0,5 | 1:2 |
| 154 | 150 | 50 | 10 | | | 0,25 | 1:2 |
| 155 | 150 | 50 | 0 | | | 0,25 | 1:2 |
| 156 | 150 | 50 | 10 | | | 0,1 | 1:2 |
| 157 | 150 | 50 | 0 | | | 0,1 | 1:2 |
| 158 | 150 | 50 | 10 | | | 0,5 | 0,5:2,5 |
| 159 | 150 | 50 | 0 | | | 0,5 | 0,5:2,5 |
| 160 | 150 | 50 | 10 | | | 0,25 | 0,5:2,5 |
| 161 | 150 | 50 | 0 | | | 0,25 | 0,5:2,5 |
| 162 | 150 | 50 | 10 | | | 0,1 | 0,5:2,5 |
| 163 | 150 | 50 | 0 | | | 0,1 | 0,5:2,5 |
| 176 | 150 | 50 | 10 | 0,5 | | | 1:2 |
| 177 | 150 | 50 | 0 | 0,5 | | | 1:2 |
| 178 | 150 | 50 | 10 | 0,25 | | | 1:2 |
| 179 | 150 | 50 | 0 | 0,25 | | | 1:2 |
| 180 | 150 | 50 | 10 | 0,1 | | | 1:2 |
| 181 | 150 | 50 | 0 | 0,1 | | | 1:2 |
| 182 | 150 | 50 | 10 | 0,5 | | | 0,5:2,5 |
| 183 | 150 | 50 | 0 | 0,5 | | | 0,5:2,5 |
| 184 | 150 | 50 | 10 | 0,25 | | | 0,5:2,5 |
| 185 | 150 | 50 | 0 | 0,25 | | | 0,5:2,5 |
| 186 | 150 | 50 | 10 | 0,1 | | | 0,5:2,5 |
| 187 | 150 | 50 | 0 | 0,1 | | | 0,5:2,5 |
| 176 | 150 | 50 | 10 | | 0,5 | | 1:2 |
| 177 | 150 | 50 | 0 | | 0,5 | | 1:2 |
| 178 | 150 | 50 | 10 | | 0,25 | | 1:2 |
| 179 | 150 | 50 | 0 | | 0,25 | | 1:2 |

(fortgesetzt)

| Formulie rung | Konzentration im Solvent (85:15 EtOH:H2O) [mg/mL] | | Non-solvent (mg/mL) | | | | | Verhältnis Solvent/Non-Solvent |
|---|---|---|---|---|---|---|---|---|
| | Lipoid S100 | Wirkstoff | Natriumoleat | Tween 20 | Tween 60 | Tween 80 | | |
| 180 | 150 | 50 | 10 | | 0,1 | | | 1:2 |
| 181 | 150 | 50 | 0 | | 0,1 | | | 1:2 |
| 182 | 150 | 50 | 10 | | 0,5 | | | 0,5:2,5 |
| 183 | 150 | 50 | 0 | | 0,5 | | | 0,5:2,5 |
| 184 | 150 | 50 | 10 | | 0,25 | | | 0,5:2,5 |
| 185 | 150 | 50 | 0 | | 0,25 | | | 0,5:2,5 |
| 186 | 150 | 50 | 10 | | 0,1 | | | 0,5:2,5 |
| 187 | 150 | 50 | 0 | | 0,1 | | | 0,5:2,5 |

Die durchschnittliche Partikelgröße und Größenverteilung wurde analysiert und die Ergebnisse sind in Tabelle 15 dargestellt:

Tabelle 15.

| Formulierung | durchschnittliche Partikelgröße (nm) | PDI |
|---|---|---|
| 152 | 244,8 | 0,263 |
| 153 | 196,6 | 0,227 |
| 154 | 249,2 | 0,263 |
| 155 | 206,9 | 0,256 |
| 156 | 259,9 | 0,303 |
| 157 | 199,6 | 0,203 |
| 158 | 246,3 | 0,366 |
| 159 | 767 | 0,313 |
| 160 | 231,8 | 0,424 |
| 161 | 323,1 | 0,452 |
| 162 | 228,9 | 0,392 |
| 163 | 293,8 | 0,392 |
| 176 | 245,2 | 0,199 |
| 177 | 236,2 | 0,261 |
| 178 | 227,5 | 0,231 |
| 179 | 199 | 0,219 |
| 180 | 229 | 0,192 |
| 181 | 192,1 | 0,157 |
| 182 | 214,8 | 0,39 |
| 183 | 322,4 | 0,479 |
| 184 | 221,5 | 0,363 |
| 185 | 292,7 | 0,374 |
| 186 | 225,9 | 0,305 |

(fortgesetzt)

| Formulierung | durchschnittliche Partikelgröße (nm) | PDI |
|---|---|---|
| 187 | 292,7 | 0,318 |
| 176 | 239,1 | 0,242 |
| 177 | 202,9 | 0,233 |
| 178 | 233,3 | 0,237 |
| 179 | 190,7 | 0,234 |
| 180 | 252,5 | 0,237 |
| 181 | 196,2 | 0,219 |
| 182 | 223,6 | 0,304 |
| 183 | 352,4 | 0,519 |
| 184 | 226,2 | 0,292 |
| 185 | 312,7 | 0,284 |
| 186 | 241 | 0,372 |
| 187 | 301,6 | 0,412 |

Beispiel 11:

Weitere Nanopartikel mit dem Wirkstoff wie in Beispiel 7 wurden erfindungsgemäß hergestellt. Tabelle 16 zeigt die Versuchsbedingungen:

Tabelle 16.

| Formulierung | Konzentration im Solvent (85:15 EtOH:H2O) [mg/mL] | | | | Non-Solvent | Verhältnis Solvent/Non-Solvent |
|---|---|---|---|---|---|---|
| | Wirkstoff | Vitamin E | Desoxycholsäure | Lipoid 5100 | | |
| 395 | 50 | 2,5 | 10 | 50,0 | HCl pH3.5 | 0,3 |
| 399 | | 2,5 | 2,5 | 10,0 | | |
| 400 | | 30,0 | 50,0 | 5,0 | | |

Die durchschnittliche Partikelgröße und Größenverteilung wurde analysiert und die Ergebnisse sind in Tabelle 17 dargestellt:

Tabelle 17.

| Formulierung | durchschnittliche Partikelgröße [nm] | PDI |
|---|---|---|
| 395 | 397,4 | 0,188 |
| 399 | 322,9 | 0,505 |
| 400 | 471,9 | 1,000 |

Beispiel 12:

Weitere Nanopartikel mit dem Wirkstoff wie in Beispiel 7 wurden erfindungsgemäß hergestellt. Tabelle 18 zeigt die Versuchsbedingungen:

Tabelle 18.

| Formulierung | Konzentration im Solvent (85:15 EtOH:H2O) [mg/mL] | | | Konzentration im Non-Solvent [mg/mL] | | Solvent/Non-Solvent Ratio |
|---|---|---|---|---|---|---|
| | Wirkstoff | Vitamin E | Lipoid 5100 | Non-Solvent | Natriumdesoxycholat | |
| 383 | 50 | 3,0 | 150 | HCl pH3.5 | 25,0 | 0,5 |

Die durchschnittliche Partikelgröße und Größenverteilung wurde analysiert und die Ergebnisse sind in Tabelle 19 dargestellt:

Tabelle 19.

| Formulierung | durchschnittliche Partikelgröße [nm] | PDI | Freier Wirkstoff (%) |
|---|---|---|---|
| 383 | 527,7 | 0,304 | 4,5 |

Beispiel 13:

Weitere Nanopartikel mit dem Wirkstoff wie in Beispiel 7 wurden erfindungsgemäß hergestellt. Tabelle 20 zeigt die Versuchsbedingungen:

Tabelle 20.

| Formulierung | Konzentration im Solvent (85:15 EtOH:H2O) [mg/mL] | | | Konzentration im Non-Solvent [mg/mL] | | Verhältnis Solvent/Non-Solvent |
|---|---|---|---|---|---|---|
| | Wirkstoff | Vitamin E | Lipoid 5100 | Non-Solvent | Natriumoleat | |
| 506 | 50 | 2,5 | 200 | Wasser | 5,0 | 0,5 |
| 507 | | 2,5 | 250 | | 5,0 | |
| 508 | | 5,0 | 200 | | 5,0 | |
| 509 | | 5,0 | 250 | | 5,0 | |
| 510 | | 2,5 | 200 | | 10,0 | |
| 511 | | 2,5 | 250 | | 10,0 | |
| 512 | | 5,0 | 200 | | 10,0 | |
| 513 | | 5,0 | 250 | | 10,0 | |

Die durchschnittliche Partikelgröße und Größenverteilung wurde analysiert und die Ergebnisse sind in Tabelle 21 dargestellt:

Tabelle 21.

| Formulierung | durchschnittliche Partikelgröße [nm] | PDI | Freier Wirkstoff (%) |
|---|---|---|---|
| 506 | 520,6 | 0,464 | 5,7 |
| 507 | 519,0 | 0,659 | 4,5 |
| 508 | 574,4 | 0,381 | 4,8 |
| 509 | 571,5 | 0,408 | 3,0 |
| 510 | 347,0 | 0,367 | 0,8 |
| 511 | 373,2 | 0,451 | 0,7 |
| 512 | 307,8 | 0,359 | 0,7 |

(fortgesetzt)

| Formulierung | durchschnittliche Partikelgröße [nm] | PDI | Freier Wirkstoff (%) |
|---|---|---|---|
| 513 | 388,5 | 0,381 | 0,3 |

Beispiel 14:

Weitere Nanopartikel mit dem Wirkstoff wie in Beispiel 7 wurden erfindungsgemäß hergestellt. Tabelle 22 zeigt die Versuchsbedingungen:

Tabelle 22.

| Formulierung | Konzentration im Solvent (85:15 EtOH:H2O) [mg/mL] | | | Konzentration im Non-Solvent (Water) [mg/mL] | | Verhältnis Solvent/Non-Solvent |
|---|---|---|---|---|---|---|
| | Wirkstoff | Vitamin E | Lipoid 5100 | Natriumdesoxycholat | Natriumoleat | |
| 492 | 50 | 2,5 | 50 | 25,0 | 5,0 | 0,5 |
| 493 | | | 100 | | | |
| 494 | | | 150 | | | |
| 504 | | | 200 | | | |
| 505 | | | 250 | | | |

Die durchschnittliche Partikelgröße und Größenverteilung wurde analysiert und die Ergebnisse sind in Tabelle 23 dargestellt:

Tabelle 23.

| Formulierung | durchschnittliche Partikelgröße [nm] | PDI | Freier Wirkstoff (%) |
|---|---|---|---|
| 492 | 834,3 | 0,675 | - |
| 493 | 691,4 | 0,069 | 23,7 |
| 494 | 501,3 | 0,201 | 24,6 |
| 504 | 475,1 | 0,084 | 38,0 |
| 505 | 504,2 | 0,627 | 25,4 |

**Patentansprüche**

1.  Verfahren zur Herstellung eines Trägersystems, wobei das Trägersystem einen pharmazeutischen Wirkstoff und mindestens ein Glycerophospholipid enthält und wobei das Verfahren die Schritte umfasst:

    - Bereitstellen eines Lösungsmittelstromes, enthaltend ein organisches Lösungsmittel, das mindestens eine Glycerophospholipid und den pharmazeutischen Wirkstoff,
    - Bereitstellen eines wässrigen Stromes, und
    - Mischen des Lösungsmittelstromes und des wässrigen Stromes durch frontales Kollidieren, wodurch das Trägersystem als Nanopartikel ausgefällt wird,

    **dadurch gekennzeichnet, dass** die durch dynamische Lichtstreuung (DLS) bei einer Temperatur von 25 °C gemessene durchschnittliche Partikelgröße der ausgefällten Nanopartikel 10 bis 500 nm beträgt, der Lösungsmittelstrom 1 bis 1000 mg/mL des mindestens einen Glycerophospholipides enthält und der hydrodynamische Druck in dem wässrigen Strom mindestens 1 bar beträgt.

2.  Verfahren zumindest nach Anspruch 1, **dadurch gekennzeichnet, dass** die durch dynamische Lichtstreuung (DLS) bei einer Temperatur von 25 °C gemessene durchschnittliche Partikelgröße der ausgefällten Nanopartikel 25 bis 500 nm, bevorzugt 50 bis 250 nm, 70 bis 170 nm, 80 bis 150 nm, oder 90 bis 110 nm, beträgt.

3. Verfahren zumindest nach Anspruch 1 oder 2, **dadurch gekennzeichnet, dass** der hydrodynamische Druck in dem wässrigen Strom mindestens 2 bar beträgt, bevorzugt 2 bar bis 60 bar.

4. Verfahren zumindest nach einem der vorherigen Ansprüche, **dadurch gekennzeichnet, dass** der hydrodynamische Druck in dem Lösungsmittelstrom mindestens 0.01 bar beträgt, bevorzugt mehr als 0.1 bar, bevorzugter mehr als 1 bar, noch bevorzugter zwischen 2 bar bis 60 bar.

5. Verfahren zumindest nach einem der vorherigen Ansprüche, **dadurch gekennzeichnet, dass** die durchschnittliche Partikelgröße der ausgefällten Nanopartikel durch den hydrodynamischen Druck in dem wässrigen Strom und/oder den hydrodynamischen Druck in dem Lösungsmittelstrom eingestellt wird, insbesondere bei Konstanthalten des Mischungsverhältnisses von wässriger Phase und Lösungsmittel-Phase, wobei durch einen höheren hydrodynamischen Druck Nanopartikel geringerer durchschnittlicher Partikelgröße ausgefällt werden.

6. Verfahren zumindest nach einem der vorherigen Ansprüche, **dadurch gekennzeichnet, dass** das mindestens eine Glycerophospholipid ausgewählt ist aus Phosphatidylcholinen.

7. Verfahren zumindest nach einem der vorherigen Ansprüche, **dadurch gekennzeichnet, dass** der Lösungsmittelstrom 50 bis 700 mg/mL, 100 bis 500 mg/mL, 100 bis 300 mg/mL, oder etwa 150 oder 225 mg/mL, des mindestens einen Glycerophospholipides enthält.

8. Verfahren zumindest nach einem der vorherigen Ansprüche, **dadurch gekennzeichnet, dass** der Lösungsmittelstrom und/oder der wässrige Strom 1 bis 250 mg/mL, bevorzugt 5 bis 100 mg/mL, besonders bevorzugt 8 bis 50 mg/mL, eines Steroides, vorzugsweise Cholesterol oder eines seiner Derivate, enthält.

9. Verfahren zumindest nach einem der vorherigen Ansprüche, **dadurch gekennzeichnet, dass** das Mischen des Lösungsmittelstromes und des wässrigen Stromes ein Kollidieren der Ströme umfasst, vorzugsweise in einem Volumenverhältnis von Lösungsmittelstrom zu wässrigem Strom von zwischen 1:1 bis 1:20, bevorzugt von zwischen 1:1 bis 1:10.

10. Verfahren zumindest nach einem der vorherigen Ansprüche, **dadurch gekennzeichnet, dass** der wässrige Strom 0.1 bis 250 mg/mL, bevorzugt 0.5 bis 100 mg/mL, besonders bevorzugt 1.0 bis 15 mg/mL, eines oder mehrerer ionischer oder nichtionischer Tenside enthält, wobei das Tensid vorzugsweise Natriumoleat und/oder Ölsäure ist.

11. Verfahren zumindest nach einem der vorherigen Ansprüche, **dadurch gekennzeichnet, dass** der Lösungsmittelstrom 0.1 bis 250 mg/mL, bevorzugt 0.5 bis 100 mg/mL, besonders bevorzugt 1.0 bis 15 mg/mL, eines oder mehrerer ionischer oder nichtionischer Tenside enthält, wobei das Tensid vorzugsweise Natriumoleat und/oder Ölsäure ist.

12. Verfahren zumindest nach einem der vorherigen Ansprüche, **dadurch gekennzeichnet, dass** das organische Lösungsmittel ein mit Wasser mischbares Lösungsmittel umfasst, wobei das organische Lösungsmittel vorzugsweise i) Ethanol oder ii) eine Mischung von Ethanol mit einem weiteren organischen Lösungsmittel, das mit Wasser und Ethanol mischbar ist, ist.

13. Verfahren zumindest nach Anspruch 12, **dadurch gekennzeichnet, dass** das weitere organische Lösungsmittel Aceton ist.

14. Verfahren zumindest nach einem der vorherigen Ansprüche, **dadurch gekennzeichnet, dass** der wässrige Strom 1 bis 1000 mM (Säure und konjugierte Base), bevorzugt 20 bis 500 mM (Säure und konjugierte Base), eines Puffersystems enthält.

15. Verfahren zumindest nach einem der vorherigen Ansprüche, **dadurch gekennzeichnet, dass** die Konzentration (mg/mL) an pharmazeutischen Wirkstoff in dem Lösungsmittelstrom geringer ist als die Konzentration (mg/mL) des Glycerophospholipides, wobei der Lösungsmittelstrom vorzugsweise 0,25 bis 250 mg/mL, bevorzugt 1 bis 150 mg/mL, bevorzugt 10 bis 100 mg/mL, des pharmazeutischen Wirkstoffes enthält.

**Claims**

1. A process for the preparation of a carrier system, wherein the carrier system comprises a pharmaceutically active agent and at least one glycerophospholipid, the process comprising the steps of:

   - providing a solvent stream comprising an organic solvent, the at least one glycerophospholipid and the pharmaceutically active agent,
   - providing an aqueous stream, and
   - mixing the solvent stream and the aqueous stream by frontal collision, whereby the carrier system is precipitated as nanoparticles,

   **characterized in that** the average particle size of the precipitated nanoparticles measured by dynamic light scattering (DLS) at a temperature of 25 °C is 10 to 500 nm, the solvent stream contains 1 to 1000 mg/mL of the at least one glycerophospholipid and the hydrodynamic pressure in the aqueous stream is at least 1 bar.

2. The process according at least to claim 1, **characterized in that** the average particle size of the precipitated nanoparticles measured by dynamic light scattering (DLS) at a temperature of 25 °C is 25 to 500 nm, preferably 50 to 250 nm, 70 to 170 nm, 80 to 150 nm, or 90 to 110 nm.

3. The process according to at least claim 1 or 2, **characterized in that** the hydrodynamic pressure in the aqueous stream is at least 2 bar, preferably 2 bar to 60 bar.

4. The process according to at least one of the preceding claims, **characterized in that** the hydrodynamic pressure in the solvent stream is at least 0.01 bar, preferably greater than 0.1 bar, more preferably greater than 1 bar, even more preferably between 2 bar and 60 bar.

5. The process according to at least one of the preceding claims, **characterized in that** the average particle size of the precipitated nanoparticles is adjusted by the hydrodynamic pressure in the aqueous stream and/or the hydrodynamic pressure in the solvent stream, in particular while maintaining a constant mixing ratio of the aqueous phase and solvent phase, whereby nanoparticles of smaller average particle size are precipitated by a higher hydrodynamic pressure.

6. The process according to at least one of the preceding claims, **characterized in that** the at least one glycerophospholipid is selected from phosphatidylcholine.

7. The process according to at least one of the preceding claims, **characterized in that** the solvent stream contains 50 to 700 mg/mL, 100 to 500 mg/mL, 100 to 300 mg/mL, or about 150 or 225 mg/mL, of the at least one glycerophospholipid.

8. The process according to at least one of the preceding claims, **characterized in that** the solvent stream and/or the aqueous stream contains 1 to 250 mg/mL, preferably 5 to 100 mg/mL, more preferably 8 to 50 mg/mL, of a steroid, preferably cholesterol or one of its derivatives.

9. The process according to at least one of the preceding claims, **characterized in that** the mixing of the solvent stream and the aqueous stream comprises colliding the streams, preferably at a volume ratio of the solvent stream to the aqueous stream of between 1:1 and 1:20, preferably between 1:1 and 1:10.

10. The process according to at least one of the preceding claims, **characterized in that** the aqueous stream contains 0.1 to 250 mg/mL, preferably 0.5 to 100 mg/mL, more preferably 1.0 to 15 mg/mL, of one or more ionic or nonionic surfactants, the surfactant preferably being sodium oleate and/or oleic acid.

11. The process according to at least one of the preceding claims, **characterized in that** the solvent stream contains 0.1 to 250 mg/mL, preferably 0.5 to 100 mg/mL, more preferably 1.0 to 15 mg/mL, of one or more ionic or nonionic surfactants, the surfactant preferably being sodium oleate and/or oleic acid.

12. The process according to at least one of the preceding claims, **characterized in that** the organic solvent comprises a water-miscible solvent, wherein the organic solvent is preferably i) ethanol or ii) a mixture of ethanol with a further organic solvent which is miscible with water and ethanol.

**13.** The process at least according to claim 12, **characterized in that** the further organic solvent is acetone.

**14.** The process according to at least one of the preceding claims, **characterized in that** the aqueous stream contains 1 to 1000 mM (acid and conjugate base), preferably 20 to 500 mM (acid and conjugate base), of a buffer system.

**15.** The process according to at least one of the preceding claims, **characterized in that** the concentration (mg/mL) of the active pharmaceutical ingredient in the solvent stream is lower than the concentration (mg/mL) of the glycerophospholipid, wherein the solvent stream preferably contains 0.25 to 250 mg/mL, preferably 1 to 150 mg/mL, preferably 10 to 100 mg/mL, of the active pharmaceutical ingredient.


**Revendications**

**1.** Procédé de fabrication d'un système porteur, le système porteur contenant un principe actif pharmaceutique et au moins un glycérophospholipide et le procédé comprenant les étapes suivantes :

- fournir un flux de solvant contenant un solvant organique l'au moins un glycérophospholipide et le principe actif pharmaceutique,
- fournir un flux aqueux, et
- mélanger le flux de solvant et le flux aqueux par collision frontale, ce qui précipite le système porteur sous forme de nanoparticules,

**caractérisé en ce que** la taille moyenne des nanoparticules précipitées, mesurée par diffusion dynamique de la lumière (DLS) à une température de 25 °C, est de 10 à 500 nm, le flux de solvant contient 1 à 1000 mg/mL de l'au moins un glycérophospholipide et la pression hydrodynamique dans le courant aqueux est d'au moins 1 bar.

**2.** Procédé au moins selon la revendication 1, **caractérisé en ce que** la taille moyenne des nanoparticules précipitées, mesurée par diffusion dynamique de la lumière (DLS) à une température de 25 °C, est de 25 à 500 nm, de préférence de 50 à 250 nm, de 70 à 170 nm, de 80 à 150 nm ou de 90 à 110 nm.

**3.** Procédé au moins selon la revendication 1 ou 2, **caractérisé en ce que** la pression hydrodynamique dans le flux aqueux est d'au moins 2 bars, de préférence de 2 bars à 60 bars.

**4.** Procédé selon l'une au moins des revendications précédentes, **caractérisé en ce que** la pression hydrodynamique dans le flux de solvant est d'au moins 0,01 bar, de préférence supérieure à 0,1 bar, plus préférablement supérieure à 1 bar, encore plus préférablement comprise entre 2 bars et 60 bars.

**5.** Procédé selon l'une au moins des revendications précédentes, **caractérisé en ce que** la taille moyenne des nanoparticules précipitées est ajustée par la pression hydrodynamique dans le flux aqueux et/ou la pression hydrodynamique dans le flux de solvant, notamment **en ce que** le rapport de mélange de la phase aqueuse et de la phase solvant est maintenu constant, les nanoparticules de taille moyenne plus petite étant précipitées par une pression hydrodynamique plus élevée.

**6.** Procédé selon l'une au moins des revendications précédentes, **caractérisé en ce que** 'au moins un glycérophospholipide est choisi parmi les phosphatidylcholines.

**7.** Procédé selon l'une au moins des revendications précédentes, **caractérisé en ce que** le flux de solvant contient 50 à 700 mg/mL, 100 à 500 mg/mL, 100 à 300 mg/mL, ou environ 150 ou 225 mg/mL d'au moins un glycérophospholipide.

**8.** Procédé selon l'une au moins des revendications précédentes, **caractérisé en ce que** le flux de solvant et/ou le flux aqueux contient 1 à 250 mg/mL, de préférence 5 à 100 mg/mL, de manière particulièrement préférée 8 à 50 mg/mL, d'un stéroïde, de préférence du cholestérol ou l'un de ses dérivés.

**9.** Procédé selon l'une au moins des revendications précédentes, **caractérisé en ce que** le mélange du flux de solvant et du flux aqueux comprend la collision des flux, de préférence dans un rapport volumique du flux de solvant au flux aqueux compris entre 1:1 et 1:20, de préférence entre 1:1 et 1:10.

**10.** Procédé selon l'une au moins des revendications précédentes, **caractérisé en ce que** le flux aqueux contient 0,1 à 250 mg/mL, de préférence 0,5 à 100 mg/mL, de manière particulièrement préférée 1,0 à 15 mg/mL, d'un ou plusieurs tensioactifs ioniques ou non ioniques, le tensioactif étant de préférence l'oléate de sodium et/ou l'acide oléique.

**11.** Procédé selon l'une au moins des revendications précédentes, **caractérisé en ce que** le flux de solvant contient 0,1 à 250 mg/mL, de préférence 0,5 à 100 mg/mL, de manière particulièrement préférée 1,0 à 15 mg/mL, d'un ou plusieurs tensioactifs ioniques ou non ioniques, le tensioactif étant de préférence l'oléate de sodium et/ou l'acide oléique.

**12.** Procédé selon l'une au moins des revendications précédentes, **caractérisé en ce que** le solvant organique comprend un solvant miscible à l'eau, le solvant organique étant de préférence i) de l'éthanol ou ii) un mélange d'éthanol à un autre solvant organique miscible à l'eau et à l'éthanol.

**13.** Procédé au moins selon la revendication 12, **caractérisé en ce que** l'autre solvant organique est l'acétone.

**14.** Procédé selon l'une au moins des revendications précédentes, **caractérisé en ce que** le flux aqueux contient 1 à 1000 mM (acide et base conjuguée), de préférence 20 à 500 mM (acide et base conjuguée), d'un système tampon.

**15.** Procédé selon l'une au moins des revendications précédentes, **caractérisé en ce que** la concentration (mg/mL) du principe actif pharmaceutique dans le flux de solvant est inférieure à la concentration (mg/mL) du glycérophospholipide, le flux de solvant contenant de préférence 0,25 à 250 mg/mL, de préférence 1 à 150 mg/mL, de préférence 10 à 100 mg/mL, du principe actif pharmaceutique.

Zeichnungen

Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 2395978 B1
- EP 1165224 B1
- WO 2016146516 A1
- WO 2018234217 A1

### In der Beschreibung aufgeführte Nicht-Patentliteratur

- **PRAMANICK, S. ; SINGODIA, D. ; CHANDEL, V.** Excipient Selection In Parenteral Formulation Development. *Pharma Times,* 2013, vol. 45 (3), 65-77
- **BITTNER, B. ; MOUNTFIELD, R. J.** Intravenous administration of poorly soluble new drug entities in early drug discovery: the potential impact of formulating on pharmacokinetic parameters. *Curr. Opinion Drug Discov. Dev.,* 2002, vol. 5, 59-71
- **THEIS, J. G. et al.** Anaphylactoid reactions in children receiving high-dose intravenous cyclosporine for reversal of tumor resistance: the causative role of improper dissolution of cremophor EL. *J. Clin. Oncol.,* 1995, vol. 13, 2508-2516
- **O'DWYER, P. J. ; WEISS, R. B.** Hypersensitivity reactions induced by etoposide. *Cancer Res.,* 1984, vol. 68, 959-961
- **DUMA, R. J. ; AKERS, M. J. ; TURCO, S. J.** Pharmaceutical Dosage Forms: Parenteral Medications. Marcel Dekker, 1992, vol. 1, 17-58
- **WARD, G. H. ; YALKOWSKY, S. H.** Studies in Phlebitis. VI. Dilution-induced precipitation of amiodarone HCl. *J. Parenter. Sci. Technol.,* 1993, vol. 47, 161-165
- **DAVIO, S. R. et al.** Precipitation of the renin inhibitor ditekiren upon iv infusion; in vitro studies and their relationship to in vivo precipitation in the cynomolgus monkey. *Pharm. Res.,* 1991, vol. 8, 80-83
- **WONG, J. ; BRUGGER, A. ; KHARE, A. ; CHAUBAL, M. ; PAPADOPOULOS, P. ; RABINOW, B. E. ; NING, J.** Suspensions for intravenous (IV) injection: a review of development, preclinical and clinical aspects. *Advanced Drug Delivery Reviews,* 2008, vol. 60 (8), 939-954
- **FANG, J. ; NAKAMURA, H. ; MAEDA, H.** The EPR Effect: Unique Features of Tumor Blood Vessels for Drug Delivery, Factors Involved, and Limitations and Augmentation of the Effect. *Adv. Drug Delivery Rev.,* 2011, vol. 63, 136-151
- **RABINOW, B. E.** Nanosuspensions in drug delivery. *Nature Reviews. Drug Discovery,* 2004, vol. 3 (9), 785-796